# EUROPEAN PATENT APPLICATION

(11) **EP 4 835 050 A1**
(43) Date of publication of application: **04.11.2026**
(21) Application number: 24913628.4
(22) Date of filing: 19.12.2024
(51) Int. Cl.: C12N 1/20, A61K 35/744, A61P 25/28, A23L 33/135, A23K 10/18, C12R 1/01

(54) **LEUCONOSTOC FALKENBERGENSE LBMB321010 STRAIN, AND COMPOSITION COMPRISING SAME FOR PREVENTING, ALLEVIATING OR TREATING DEGENERATIVE BRAIN DISEASES**

(30) Priority: 26.12.2023 KR 20230191420
(71) Applicant: Liscure Bio Inc., Seongnam-si, Gyeonggi-do 13488 (KR); Celltrion, Inc., Incheon 22014 (KR)
(72) Inventor: CHIN, Hwa Sup, Yongin-si, Gyeonggi-do 16824 (KR); BYUN, Seo Hyun, Incheon 22014 (KR); SHIN, Bo Ram, Incheon 22014 (KR); LIM, Joo Hyuck, Incheon 22014 (KR); JEON, Young Hyun, Incheon 22014 (KR)
(74) Representative: Cabinet Becker et Associés
(86) International application number: PCT/KR2024/020751
(87) International publication number: WO 2025/143685

(57) **Abstract**

The present disclosure relates to a *Leuconostoc falkenbergense* LBMB321010 strain, and a composition comprising same for preventing, alleviating or treating degenerative brain diseases. The strain exhibits excellent preventive and therapeutic effects on various degenerative brain diseases, and thus may be effectively utilized in a composition for a use of treating, preventing or alleviating degenerative brain diseases in humans or animals.

## Description

### [Technical Field]

The present disclosure relates to a *Leuconostoc falkenbergense* LBMB321010 strain, and a composition including the *Leuconostoc falkenbergense* LBMB321010 strain for preventing, alleviating or treating degenerative brain diseases.

### [Background Art]

For modern people, memory has gained importance in rapidly changing life, and becomes a major subject of interest socially, ranging from adolescents with a lot of learning to the elderly. Degenerative brain diseases are known to cause memory loss, and brain inflammatory responses have been found as a significant factor in degenerative brain diseases of the central nervous system, such as Alzheimer's disease, Parkinson's disease, and Huntington's disease (Jin DQ et al, Biochemical and Biophysical Research Communications, 331: 1264-1269, 2005; Lim CS et al, Biological and Pharmaceutical Bulletin, 29: 1212-1216, 2006).

Parkinson's disease (PD) is a degenerative neurological disease that is the second most prevalent disease of degenerative brain diseases after Alzheimer's disease, and involves the progressive degeneration of dopamine-secreting neurons distributed from the substantia nigra to the striatum. Currently, dopamine alternative therapy such as L-DOPA and dopamine receptor agonists is used for the treatment of Parkinson's disease, but it is known that serious side effects, such as dyskinesia, occur with long-term administration.

Recently, since patients with degenerative brain diseases such as dementia have rapidly increased due to an increase in the elderly population, efforts have been made to establish various treatment strategies and develop effective drugs to alleviate and improve cognitive and learning functions impaired by dementia, etc. Memory-improving drugs developed to date include an acetylcholine precursor, a receptor agonist, an acetylcholine esterase inhibitor, etc. However, a therapeutic agent capable of treating the fundamental cause of degenerative brain diseases has not yet been developed, and commonly usable therapeutic agents include Pfizer's Aricept, Novartis' Exelon, and Janssen's Reminyl, which are acetylcholinesterase inhibitors, and Lundbeck's Ebixa (Memantine), which is an antagonist mechanism of a N-methyl-D-aspartate (NMDA) receptor and has been recently approved by the US FDA. However, the acetylcholinesterase inhibitors have limitations in only alleviating declining cognitive abilities, but not treating the fundamental cause of Alzheimer's disease. Furthermore, it is known that the acetylcholinesterase inhibitors exhibit a temporary symptom relief effect only in some patients, but the effect thereof does not last long, so that it is difficult to expect a fundamental therapeutic effect. In addition, due to the characteristics of degenerative brain diseases, long-term use of drugs is required, but the drugs have problems such as accompanying various side effects including liver toxicity, vomiting, and loss of appetite. Therefore, the development of novel therapeutic agents capable of preventing the progression of degenerative brain diseases is becoming an urgent task.

Meanwhile, lactic acid bacteria are widely distributed in the oral cavity, intestines, vagina, and feces of humans and animals, fermented foods such as kimchi, etc., and are closely related to the health of humans and animals. The lactic acid bacteria exhibit various health-promoting effects, such as intestinal regulation, inhibition of harmful bacteria, immunoregulation, lowered blood cholesterol, anticancer, etc. Furthermore, recent research results have found that intestinal microorganisms actively influence human intestinal health and intestinal diseases, as well as the regulation of brain function and the onset of brain diseases.

Meanwhile, Korean Patent Registration No. 10-2404016 discloses 'Lactococcus lactis subsp. hordniae strain and use of Lactococcus lactis subsp. Hordniae strain for preventing, alleviating or treating degenerative brain diseases', and Korean Patent Publication No. 10-2020-0020850 discloses 'Composition comprising bacterial strains belonging to the species lactobacillus salivarius for the treatment of Parkinson's disease'. However, no preventive or therapeutic effect on degenerative brain diseases have been found using a novel *Leuconostoc falkenbergense* strain or a culture thereof as an active ingredient.

### [Disclosure]

### [Technical Problem]

An object of the present disclosure is to provide a *Leuconostoc falkenbergense* LBMB321010 (Accession number: KCTC15613BP) strain having excellent α-synuclein aggregation inhibitory activity.

Another object of the present disclosure is to provide a pharmaceutical composition for preventing or treating degenerative brain diseases, including the strain or a culture thereof as an active ingredient.

Yet another object of the present disclosure is to provide a food composition for preventing or alleviating degenerative brain diseases, including the strain or a culture thereof as an active ingredient.

Still another object of the present disclosure is to provide a food additive composition for preventing or alleviating degenerative brain diseases, including the strain or a culture thereof as an active ingredient.

Still another object of the present disclosure is to provide a feed composition for preventing or alleviating degenerative brain diseases of animals, including the strain or a culture thereof as an active ingredient.

Still another object of the present disclosure is to provide a feed additive composition for preventing or alleviating degenerative brain diseases of animals, including the strain or a culture thereof as an active ingredient.

### [Technical Solution]

An aspect of the present disclosure provides a *Leuconostoc falkenbergense* LBMB321010 (Accession number: KCTC15613BP) strain having excellent α-synuclein aggregation inhibitory activity.

Another aspect of the present disclosure provides a pharmaceutical composition for preventing or treating degenerative brain diseases, including the strain or a culture thereof as an active ingredient.

According to an embodiment of the present disclosure, the degenerative brain disease may be any one disease selected from the group consisting of Parkinson's disease, Alzheimer's disease, Huntington's disease, amyotrophic lateral sclerosis, Creutzgeldt-Jakob disease, stroke, and multiple sclerosis.

According to an embodiment of the present disclosure, the composition may be administered orally or parenterally.

According to an embodiment of the present disclosure, the parenteral administration may be intravenous injection, subcutaneous injection, intramuscular injection, intraperitoneal injection, intradermal administration, topical administration, intranasal administration, intrapulmonary administration, or intrarectal administration.

Yet another aspect of the present disclosure provides a food composition for preventing or alleviating degenerative brain diseases, including the strain or a culture thereof as an active ingredient.

According to an embodiment of the present disclosure, the food may be a health functional food.

Still another aspect of the present disclosure provides a food additive composition for preventing or alleviating degenerative brain diseases, including the strain or a culture thereof as an active ingredient.

Still another aspect of the present disclosure provides a feed composition for preventing or alleviating degenerative brain diseases of animals, including the strain or a culture thereof as an active ingredient.

Still another aspect of the present disclosure provides a feed additive composition for preventing or alleviating degenerative brain diseases of animals, including the strain or a culture thereof as an active ingredient.

### [Advantageous Effects]

According to the present disclosure, the *Leuconostoc falkenbergense* LBMB321010 strain and the composition including the same for preventing, alleviating or treating degenerative brain diseases exhibit excellent preventive and therapeutic effects on various degenerative brain diseases, and thus may be effectively utilized as a composition for treating, preventing or alleviating degenerative brain diseases in humans or animals.

### [Description of drawings]

FIG. 1 is a diagram showing results taken by using fluorescence microscopy in order to confirm an α-synuclein aggregation inhibitory effect after inducing α-synuclein aggregation and then treating a composition according to the present disclosure, and a graph showing calculated ratios of GFP fluorescence value areas to RFP.
FIG. 2 is a graph showing results of measuring TNFα secretion in order to confirm the degree of inhibition of inflammatory factors by treatment with the composition according to the present disclosure.
FIG. 3 is a graph showing results of performing a pole test, in order to confirm the degree of alleviating motor ability in a Parkinson's mouse model by treatment with the composition according to the present disclosure.
FIG. 4 is a diagram and a graph showing a relative expression level of α-synuclein protein to tubulin, in order to confirm the degree of inhibiting α-synuclein in a Parkinson's mouse model by treatment with the composition according to the present disclosure.
FIG. 5 is a diagram showing results of staining brain tissue with tyrosine hydroxylase (TH) and a graph calculating and showing the number of dopamine neurons in the substantia nigra pars compacta of the midbrain, in order to confirm the degree of death inhibition of dopamine neurons in a Parkinson's mouse model by treatment with the composition according to the present disclosure.
FIG. 6 is a graph showing results of measuring fluorescence values according to MAO-B activity, in order to confirm the degree of inhibition of MAO-B activity in a Parkinson's mouse model by treatment with the composition according to the present disclosure.

### [Best Mode]

An aspect of the present disclosure provides a *Leuconostoc falkenbergense* LBMB321010 (Accession number: KCTC15613BP) strain having excellent α-synuclein aggregation inhibitory activity.

The *Leuconostoc falkenbergense* LBMB321010 strain according to the present disclosure was isolated from kimchi and was deposited at the Korean Collection for Type Cultures (KCTC) of the Korea Research Institute of Bioscience and Biotechnology on September 14, 2023 (Accession No.: KCTC15613BP).

The *Leuconostoc falkenbergense* LBMB321010 strain has a 16S rRNA gene nucleic acid sequence of SEQ ID NO: 1 (Table 1).

**[Table 1]**

| |
|---|
| DNA sequence (5'→3') of 16S rRNA gene of *Leuconostoc falkenbergense* LBMB321010 |
| agtgacaggtggtgcatggtcgtcgtcagctcgtgtcgtgagatgttgggttaagtcccgcaacgagcgcaacccttattgttagttgcca gcattcagatgggcactctagcgagactgccggtgacaaaccggaggaaggcggggacgacgtcagatcatcatgccccttatgacc tgggctacacacgtgctacaatggcgtatacaacgagttgccaacccgcgagggtgagctaatctcttaaagtacgtctcagttcggatt gtagtctgcaactcgactacatgaagtcggaatcgctagtaatcgcggatcagcacgccgcggtgaatacgttcccgggtcttgtacaca ccgcccgtcacaccatgggagtttgtaatgcccaaagccggtggcctaaccttttaggaaggagccgtctaaggcaggacagatgact ggggtg |

0.1 to 10% of the *Leuconostoc falkenbergense* LBMB321010 strain may be inoculated into a MRS liquid medium and then cultured and used at 25 to 37°C for 4 to 48 hours.

The culturing method is preferably a static culture method, but is not limited thereto.

Another aspect of the present disclosure provides a pharmaceutical composition for preventing or treating degenerative brain diseases, including the strain or a culture thereof as an active ingredient.

The composition including the *Leuconostoc falkenbergense* LBMB321010 strain of the present disclosure or the culture thereof as the active ingredient has a preventive or therapeutic effect on degenerative brain diseases, and may be used as a pharmaceutical composition.

The *Leuconostoc falkenbergense* LBMB321010 strain of the present disclosure is a lactic acid bacteria strain. The *Leuconostoc falkenbergense* LBMB321010 strain is probiotics, and has general intestinal regulating and immune boosting effects of lactic acid bacteria. It is a well-known fact that Leuconostoc sp. lactic acid bacteria have an intestinal regulating effect and an immune boosting effect.

In the present disclosure, the "probiotics" are understood as the meaning of a living microorganism that has a beneficial effect on the health of a host by improving an intestinal microbial environment of the host in the gastrointestinal tract of animals, including humans. The probiotics are living microorganisms with probiotic activity, which may have a beneficial effect on the intestinal flora of the host, when fed to humans or animals in the form of dried cells or fermentation products in the form of single or combined strains.

According to an embodiment of the present disclosure, the degenerative brain disease may be any one disease selected from the group consisting of Parkinson's disease, Alzheimer's disease, Huntington's disease, amyotrophic lateral sclerosis, Creutzgeldt-Jakob disease, stroke, and multiple sclerosis.

The *Leuconostoc falkenbergense* LBMB321010 strain included in the composition according to the present disclosure may exist as live or dead cells, and may also exist in a dried or freeze-dried form. In addition, a culture of the *Leuconostoc falkenbergense* LBMB321010 strain may be an active ingredient, and the culture may include a live cell culture medium or a dead cell supernatant. Types of lactic acid bacteria suitable to be included in various compositions and formulation methods are well known to those skilled in the art.

According to an embodiment of the present disclosure, the composition may be administered orally or parenterally.

According to an embodiment of the present disclosure, the parenteral administration may be intravenous injection, subcutaneous injection, intramuscular injection, intraperitoneal injection, intradermal administration, topical administration, intranasal administration, intrapulmonary administration, or intrarectal administration.

The composition may be administered orally or parenterally. In the case of the parenteral administration, the composition may be administered by intravenous injection, subcutaneous injection, intramuscular injection, intraperitoneal injection, intradermal administration, topical administration, intranasal administration, intrapulmonary administration, intrarectal administration, etc., and preferably intravenous injection, but it is not limited thereto.

A suitable dose of the composition may be variously prescribed by factors, such as a formulation method, an administration type, age, weight, and sex of a patient, a pathological condition, food, an administration time, an administration route, an excretion rate, and response susceptibility.

When the composition of the present disclosure is used as a pharmaceutical composition, the pharmaceutical composition of the present disclosure may be prepared by using pharmaceutically suitable and physiologically acceptable adjuvants in addition to the active ingredient. As the adjuvant, excipients, disintegrants, sweeteners, binders, coating agents, expanding agents, lubricants, slip modifiers, or flavoring agents may be used.

The pharmaceutical composition may be preferably formulated by further including at least one pharmaceutically acceptable carrier in addition to the above-described active ingredient for administration.

For example, for formulation in the form of tablets or capsules, the active ingredient may be combined with a non-toxic pharmaceutically acceptable inactive carrier, such as ethanol, glycerol, water, or the like. Further, if desired or necessary, suitable binders, lubricants, disintegrants and coloring agents may also be included as a mixture. The suitable binder is not limited thereto, but includes natural sugars such as starch, gelatin, glucose or beta-lactose, corn sweeteners, natural and synthetic gums such as acacia, tragacanth or sodium oleate; sodium stearate, magnesium stearate, sodium benzoate, sodium acetate, sodium chloride and the like. The disintegrant is not limited thereto, but includes starch, methylcellulose, agar, bentonite, xanthan gum, or the like. In the composition formulated with a liquid solution, the pharmaceutically acceptable carrier is suitable for sterilization and living bodies, and may use saline, sterilized water, ringer's solution, buffered saline, albumin injection solution, dextrose solution, maltodextrin solution, glycerol, ethanol, and a mixture of at least one of these ingredients, and if necessary, may add other general additives such as antioxidants, buffers, bacteriostatic agents, and the like. In addition, the pharmaceutical composition may be formulated in injectable formulations such as an aqueous solution, a suspension, and an emulsion, pills, capsules, granules, or tablets by further adding a diluent, a dispersant, a surfactant, a binder, and a lubricant.

Furthermore, as a suitable method in the art, the pharmaceutical composition may be formulated preferably according to each disease or ingredient using a method disclosed in Remington's Pharmaceutical Science, Mack Publishing Company, Easton PA.

Yet another aspect of the present disclosure provides a food composition for preventing or alleviating degenerative brain diseases, including the strain or a culture thereof as an active ingredient.

Still another aspect of the present disclosure provides a food additive composition for preventing or alleviating degenerative brain diseases, including the strain or a culture thereof as an active ingredient.

According to an embodiment of the present disclosure, the food may be a health functional food.

The food composition may be a health functional food form.

The "functional health food" refers to food prepared and processed using raw materials or ingredients that have functionality useful to the human body in accordance with the Health Functional Food Act (Article 3 (1)), and the functionality means regulating nutrients for the structure and function of the human body or obtaining useful effects for health uses such as physiological functions (Article 3 (2)).

The food composition may further include food additives, and compliance as the food additives is determined by the specifications and standards for the corresponding item in accordance with the General Rules of the Korea Food Additives Code and general test methods approved by the Ministry of Food and Drug Safety, unless otherwise specified.

The items disclosed in the Korean Food Additives Codex may include, for example, chemical composites such as ketones, glycine, calcium citrate, nicotinic acid, cinnamic acid, and the like; natural additives such as persimmon pigment, licorice extract, crystal cellulose, guar gum, and the like; mixed preparations such as sodium L-glutamic acid preparations, alkali agents added to noodles, preservative preparations, tar color preparations, etc.

Foods including the active ingredient of the present disclosure may include confectionery such as bread, rice cakes, dried fruits and nuts, candy, chocolate, chewing gum, and jam; ice cream products such as ice cream, frozen confectionery, and ice cream powder; dairy products such as milk, low-fat milk, lactose-degraded milk, processed milk, goat milk, fermented milk, butter milk, concentrated milk, milk cream, butter milk, natural cheese, processed cheese, powdered milk, and whey; meat products such as processed meat products, processed egg products, and hamburgers; fish meat products such as fish cake, ham, sausage, bacon, etc.; noodles such as ramen, dried noodles, fresh noodles, fried noodles, gelatinized dried noodles, improved cooked noodles, frozen noodles, and pasta; drinks such as vegetable drinks, carbonated drinks, soy milk, lactic acid bacteria drinks such as yogurt, and mixed drinks; seasoned foods such as soy sauce, soybean paste, gochujang, chunjang, cheonggukjang, mixed soy sauce, vinegar, sauces, tomato ketchup, curry, and dressing; margarine, shortening and pizza, but are not limited thereto.

In addition, the composition of the present disclosure may include various nutrients, vitamins, electrolytes, flavoring agents, coloring agents, pectic acid and salts thereof, alginic acid and salts thereof, organic acid, a protective colloidal thickener, a pH adjusting agent, a stabilizer, a preservative, glycerin, alcohol, a carbonic acid agent used in a carbonated drink, or the like. In addition, the composition of the present disclosure may include pulps for preparing natural fruit juices, fruit juice beverages or vegetable beverages. These ingredients may be used independently or in combination.

The beverage composition including the active ingredient of the present disclosure is not particularly limited to other ingredients, and may contain various flavoring agents, natural carbohydrates, or the like as additional ingredients like conventional beverages. Examples of the above-mentioned natural carbohydrates may include general sugars, such as monosaccharides (e.g., glucose, fructose and the like); disaccharides (e.g., maltose, sucrose and the like); and polysaccharides (e.g., dextrin, cyclodextrin and the like); and sugar alcohols such as xylitol, sorbitol, erythritol, and the like. As flavoring agents other than those described above, natural flavoring agents (thaumatin, stevia extract (e.g., rebaudioside A, glycyrrhizin), etc.) and synthetic flavoring agents (saccharine, aspartame, etc.) may be advantageously used.

Yet another aspect of the present disclosure provides a feed composition for preventing or alleviating degenerative brain diseases of animals, including the strain or a culture thereof as an active ingredient.

Still another aspect of the present disclosure provides a feed additive composition for preventing or alleviating degenerative brain diseases of animals, including the strain or a culture thereof as an active ingredient.

When the composition is prepared as a feed additive, the composition may be prepared in the form of 20 to 90% high-concentrate or powder or granules. The feed additive may further include any one or at least one of organic acids such as citric acid, fumaric acid, adipic acid, lactic acid, and malic acid; phosphates such as sodium phosphate, potassium phosphate, acidic pyrophosphate, and polyphosphate; or natural antioxidants such as polyphenol, catechin, alpha-tocopherol, rosemary extract, vitamin C, green tea extract, licorice extract, chitosan, tannic acid, phytic acid, and the like. When prepared as a feed, the composition may be prepared in a conventional feed form, and may include general feed ingredients together.

The feed and the feed additives may further include grains, such as milled or ground wheat, oats, barley, corn and rice; vegetable protein feeds, such as feeds using rape, soybean, and sunflower as a main ingredient; animal protein feeds, such as blood meal, meat meal, bone meal and fish meal; sugar and dairy products, such as dry ingredients consisting of various powdered milk and whey powder, and the like, and in addition, may further include nutritional supplements, digestion and absorption enhancers, growth promoters, and the like.

The feed additives may be administered to animals alone or also administered in combination with other feed additives in an edible carrier. In addition, the feed additives may be easily administered to animals as a top dressing, directly mixed with animal feed, or in an oral formulation separated from feed. When the feed additives are administered separately from animal feed, the feed additives may be prepared as an immediate release or sustained release formulation in combination with a pharmaceutically acceptable edible carrier, as well-known in the art. These edible carriers may be solids or liquids, for example corn starch, lactose, sucrose, soybean flakes, peanut oil, olive oil, sesame oil and propylene glycol. When the solid carrier is used, the feed additives may be tablets, capsules, powder, troches or sugar-containing tablets, or microdispersible top dressing. When the liquid carrier is used, the feed additives may be formulations of gelatin soft capsules, syrups, suspensions, emulsions, or solutions.

In addition, the feed and the feed additives may contain auxiliary agents, such as preservatives, stabilizers, wetting agents or emulsifying agents, solution accelerators, and the like. The feed additives may be added and used to animal feed by steeping, spraying or mixing.

The feed or feed additives of the present disclosure may be applied to a plurality of animal diets including mammals, poultry and fish.

The mammals may be used with pigs, cows, horses, sheep, rabbits, goats, rats, hamsters, and guinea pigs, which are rodents and laboratory rodents, as well as pets (e.g., dogs and cats), etc. The poultry may be used with chickens, turkeys, ducks, geese, pheasants, quails, etc., and the fish may be used with carp, crucian carp, trout, etc., but are not limited thereto.

### [Modes]

Hereinafter, the present disclosure will be described in more detail through one or more Examples. However, these Examples are illustrative of the present disclosure, and the scope of the present disclosure is not limited to these Examples.

### Example 1. Culture of strain

A *Leuconostoc falkenbergense* LBMB321010 strain was deposited at the Korean Collection for Type Cultures (KCTC) of the Korea Research Institute of Bioscience and Biotechnology on September 14, 2023 (Accession number KCTC15613BP).

The *Leuconostoc falkenbergense* LBMB321010 strain was inoculated with 0.1% (v/v) of strain stock in 30 ml of an MRS liquid medium and statically cultured at 30°C for 24 hours.

### Example 2. Preparation of strain culture medium

The cultured *Leuconostoc falkenbergense* LBMB321010 strain in Example 1 was washed three times with a phosphate buffered saline (PBS) solution to remove a remaining medium ingredient, divided into three equal parts, and each part was statically cultured at 30°C for 24 hours in 10 ml each of cell culture media of RPMI1640, DMEM, and MEM. After culture, only the culture media were separated by centrifugation at 3500 rpm for 10 minutes, the pH was adjusted to 7.2, and the culture media passed through a 0.2 µm filter to prepare strain culture media. The prepared strain culture media were subsequently used in the experiments of Examples 3 and 4.

### Example 3. Confirmation of α-synuclein aggregation inhibitory effect

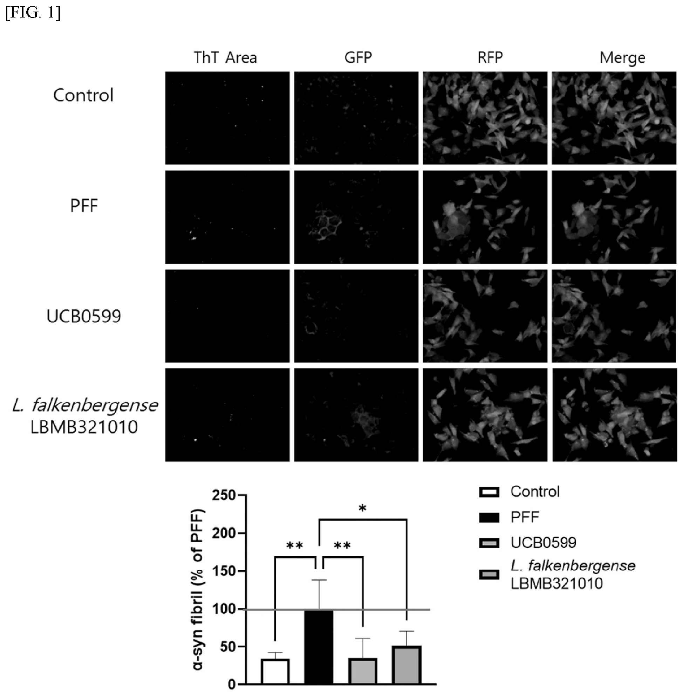

To confirm a α-synuclein aggregation inhibitory effect on a *Leuconostoc falkenbergense* LBMB321010 strain, an SH-SY5Y cell line stably expressing red fluorescent alpha-synuclein was cultured at 1 × 10⁵ cells/well in a 4-well chamber, and pre-formed fibrils (PFF) were diluted in the medium and used in 4 µg/ml to induce α-synuclein aggregation. The culture medium of the *Leuconostoc falkenbergense* LBMB321010 strain cultured in Example 2 above was diluted to be 5% of a total volume of the medium and used, and as a control group, UCB0599, an α-synuclein aggregation inhibitor, was used at 2 µM. The treated cells were cultured in a CO₂ incubator for 48 hours, reacted, and then fixed with 4% paraformaldehyde (PFA), and the nuclei of α-synuclein aggregated with thioflavin T (ThT) were stained with DAPI. GFP (ThT), RFP (a-synuclein), and DAPI were measured at a magnification of 400× using a fluorescence microscope, and the GFP and RFP fluorescence values were obtained by calculating the area of a threshold of 30 or higher through imageJ and a result of calculating a ratio of the GFP area to the RFP area was shown in FIG. 1.

As shown in FIG. 1, in a group treated with PFF, α-synuclein aggregation increased compared to the control group, and a group treated with the *Leuconostoc falkenbergense* LBMB321010 strain culture medium showed an aggregation inhibition rate of 48%. In light of the results, it was confirmed that the composition including the *Leuconostoc falkenbergense* LBMB321010 strain according to the embodiment of the present disclosure as an active ingredient or including the culture thereof as an active ingredient may alleviate a Parkinson's disease by inhibiting the α-synuclein aggregation in neurons.

### Example 4. Confirmation of inflammatory factor inhibitory effect

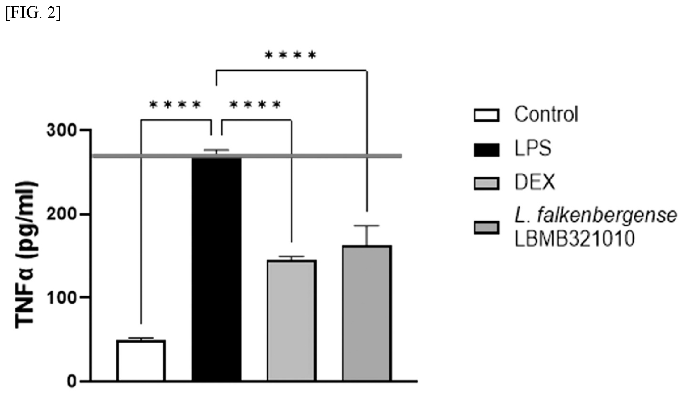

To confirm an inflammatory factor production inhibitory effect on the *Leuconostoc falkenbergense* LBMB321010 strain, a BV2 cell line was cultured at 4 × 10⁴ cells/well in a 96-well plate, and the *Leuconostoc falkenbergense* LBMB321010 strain culture medium cultured in Example 2 above was diluted to 5% of the total volume of the medium and used, and lipopolysaccharide (LPS) was diluted in the medium and used at 0.1 µg/ml. 10 nM of dexamethason (DEX) was used as a control group, and cultured in a CO₂ incubator for 24 hours to obtain the reacting medium, and TNFα was measured using a TNFα Mouse Uncoated ELISA Kit, and the results were shown in FIG. 2.

As shown in FIG. 2, it was confirmed that in the group treated with the *Leuconostoc falkenbergense* LBMB321010 strain culture medium, 39% TNFα secretion decreased compared to the LPS treated group. In light of the results, it was confirmed that the composition including the *Leuconostoc falkenbergense* LBMB321010 strain as an active ingredient or including the culture thereof as an active ingredient may alleviate a Parkinson's disease by an anti-inflammatory action.

### Example 5. Induction of Parkinson's disease using MPTP and probenecid in mouse model and administration of Leuconostoc falkenbergense strain

To confirm a preventive, alleviating, or therapeutic effect on the *Leuconostoc falkenbergense* LBMB321010 strain in a Parkinson's mouse model, 1-methyl-4-phenyl-1,2,3,6-tetrahydropyridine (MPTP) and probenecid were injected intraperitoneally into C57BL/6 mice at doses of 25 mg/kg and 250 mg/kg, respectively. The probenecid was injected 30 minutes earlier, and injected a total of 11 times twice a week. The *Leuconostoc falkenbergense* LBMB321010 strain cultured in Example 1 above was diluted to 1 × 10⁹ colony forming unit (CFU)/100 µl and administered orally 5 times a week for a total of 26 times. As a control group, an α-synuclein aggregation inhibitor, UCB0599 was injected intraperitoneally at a dose of 1 mg/kg.

### Example 6. Confirmation of motor ability improving effect in Parkinson's disease mouse model

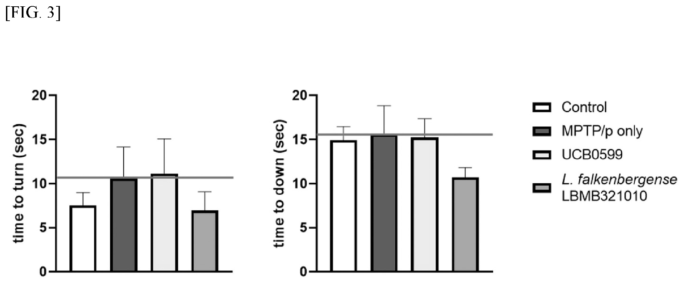

To confirm a motor ability improving effect on the *Leuconostoc falkenbergense* LBMB321010 strain in a Parkinson's mouse model, a pole test was performed on Parkinson's disease mice administered with the *Leuconostoc falkenbergense* LBMB321010 strain in Example 5. Before separating mouse groups, a pole test adaptation test was conducted, and the test was evaluated three times 2 to 3 days after the last MPTP injection. FIG. 3 shows the results of recording the time taken for the mouse to change a direction downward and the total time taken to reach the bottom of the pole after positioning the mouse upward from the top of the pole within a time limit of 30 seconds.

As shown in FIG. 3, in a group treated with MPTP and probenecid to induce Parkinson's disease, the times taken to change the direction and reach the bottom of the pole increased compared to the control group, and in a group administered with the *Leuconostoc falkenbergense* LBMB321010 strain, the direction changing time and the reaching time decreased by 34% and 31% compared to the Parkinson's disease-induced group, respectively. In light of the results, it was confirmed that the administration of the *Leuconostoc falkenbergense* LBMB321010 strain treated or alleviated Parkinson's disease to improve the motor ability.

### Example 7. Confirmation of α-synuclein inhibitory effect in Parkinson's disease mouse model

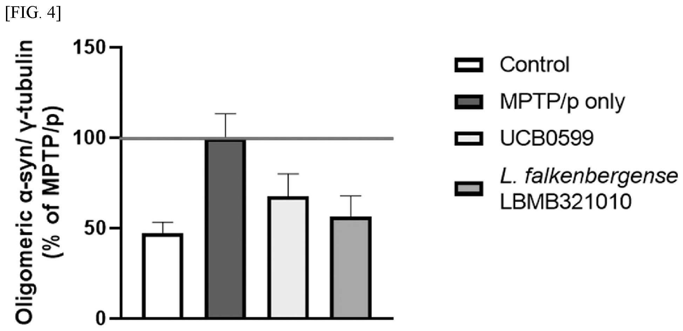

To confirm a α-synuclein inhibitory effect on a *Leuconostoc falkenbergense* LBMB321010 strain in a Parkinson's mouse model, the brain of the Parkinson's disease mouse administered with the *Leuconostoc falkenbergense* LBMB321010 strain in Example 5 was extracted, and then the substantia nigra (SN) tissue was isolated from the left brain. The tissue was added in a solution containing 1% Triton X-100, 2 mM ethylene-diamine-tetraacetic acid (EDTA), 1% protease inhibitor, and 1% phosphatase inhibitor, ground, and then sonicated and centrifuged to isolate proteins and quantify the proteins using bovine serum albumin (BSA). Equal amounts of proteins were added with a sample buffer containing sodium dodecyl sulfate (SDS) and β-mercaptoethanol, boiled at 98°C for 10 minutes, and then electrophored and transferred. The membrane was blocked with 5% skim milk, attached with a primary antibody overnight at 4°C or for 2 hours at room temperature, then washed 3 times with Tris-buffered saline with Tween 20 (TBST), and then attached with a secondary antibody for 1 hour at room temperature. Protein bands were treated with an enhanced chemiluminescence (ECL) solution and analyzed using Imagequant software. The expression value of α-synuclein for gamma-tubulin was calculated, and then the result standardized to the Parkinson's disease-induced group was shown in FIG. 4.

As shown in FIG. 4, in the group treated with MPTP and probenecid to induce Parkinson's disease, the expression of α-synuclein polymer increased by 111% compared to the control group, and in the group administered with the *Leuconostoc falkenbergense* LBMB321010 strain, the expression of α-synuclein polymer decreased by 43% compared to the Parkinson's disease-induced group. In light of the results, it was confirmed that the administration of the *Leuconostoc falkenbergense* LBMB321010 strain inhibited the α-synuclein aggregation to treat or alleviate Parkinson's disease.

### Example 8. Confirmation of effect of improving dopamine neurons in Parkinson's disease mouse model

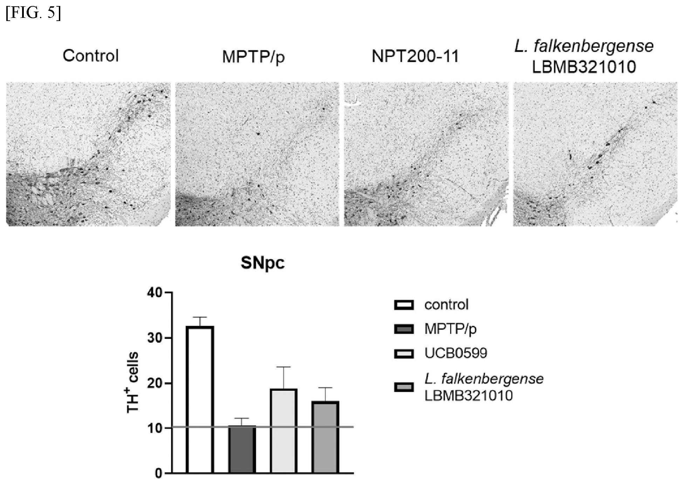

To confirm an effect of improving dopamine neurons on the *Leuconostoc falkenbergense* LBMB321010 strain in a Parkinson's mouse model, the brains of Parkinson's disease mice administered with the *Leuconostoc falkenbergense* LBMB321010 strain in Example 5 were extracted, and then the right brains were immersed in 4% paraformaldehyde and fixed at 4°C. The fixed tissue was prepared in a paraffin block, and then the substantia nigra region was attached to a slide at a thickness of 5 µm and stained with tyrosine hydroxylase as a marker of dopamine neurons. The staining results of the substantia nigra region and the number of dopamine neurons in the substantia nigra pars compacta (SNpc) were shown in FIG. 5.

As shown in FIG. 5, in the group treated with MPTP and probenecid to induce Parkinson's disease, the number of dopamine neurons in the substantia nigra pars compacta of the midbrain decreased by 67% compared to the control group, and in the group administered with the *Leuconostoc falkenbergense* LBMB321010 strain, the number of dopamine neurons increased by 49% compared to the Parkinson's disease-induced group. In light of the results, it was confirmed that the administration of the *Leuconostoc falkenbergense* LBMB321010 strain inhibited the death of dopamine neurons to treat or alleviate Parkinson's disease.

### Example 9. Confirmation of MAO-B inhibitory effect in Parkinson's disease mouse model

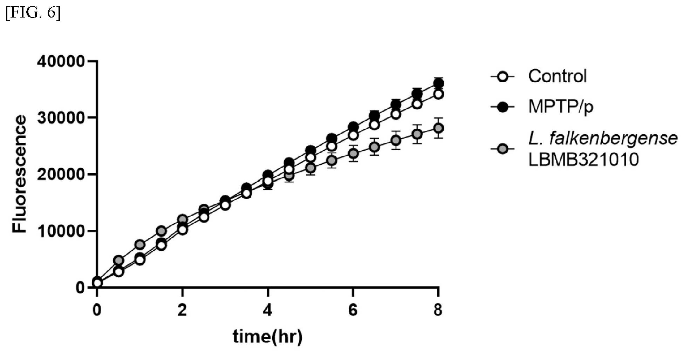

To confirm a Mao-B activity inhibitory effect on a *Leuconostoc falkenbergense* LBMB321010 strain in a Parkinson's mouse model, the brain of the Parkinson's disease mouse administered with the *Leuconostoc falkenbergense* LBMB321010 strain in Example 5 was extracted, and then the brain tissue including the hippocampous region was isolated from the left brain. The tissue was added in a solution containing 1% Triton X-100, 2 mM EDTA, and 1% protease inhibitor, ground, and then sonicated and centrifuged to isolate proteins and quantify the isolated protein with BSA. Equal amounts of proteins were reacted according to an experimental method of the Amplex Red Monoamine Oxidase Assay Kit and then fluorescence values were measured at 530 to 560 nm (excitation) and 590 nm (emission) at 30-minute intervals for 8 hours. A graph quantifying the results of measuring fluorescence values was shown in FIG. 6.

As shown in FIG. 6, in the group treated with MPTP and probenecid to induce Parkinson's disease based on 8 hours, the fluorescence values increased by 5% compared to the control group, and in the group administered with the *Leuconostoc falkenbergense* LBMB321010 strain, the fluorescence values decreased by 21% compared to the Parkinson's disease-induced group. In light of the results, it was confirmed that the administration of the *Leuconostoc falkenbergense* LBMB321010 strain inhibited the MAO-B activity to treat or alleviate Parkinson's disease.

When considering the results of Examples above, it was confirmed that the composition including the *Leuconostoc falkenbergense* LBMB321010 strain or the culture thereof as an active ingredient exhibits excellent effects on α-synuclein aggregation and reduction of MAO-BI activity in astrocytes, and thus may be utilized as a composition for preventing or treating degenerative brain diseases including Parkinson's disease.

The present disclosure has been described above with reference to the embodiments thereof. It will be understood to those skilled in the art that the present disclosure may be implemented as modified forms without departing from an essential characteristic of the present disclosure. Therefore, the disclosed embodiments should be considered in an illustrative viewpoint rather than a restrictive viewpoint. The scope of the present disclosure is defined by the appended claims rather than by the foregoing description, and all differences within the scope of equivalents thereof should be construed as being included in the present disclosure.

[Accession Number]
Depositary Authority Name: Korean Collection for Type Cultures (KCTC) of the Korea Research Institute of Bioscience and Biotechnology
Accession number: KCTC15613BP
Accession Date: 20230914

BUDAPEST TREATY ON THE INTERNATIONAL RECOGNITION OF THE DEPOSIT OF
MICROORGANISMS FOR THE PURPOSES OF PATENT PROCEDURE
INTERNATIONAL FORM
RECEIPT IN THE CASE OF AN ORIGINAL DEPOSIT
issued pursuant to Rule 7.1

TO: LISCure Biosciences Inc.
700, Daewangpangyo-ro, Bundang-gu, Seongnam-si, Gyeonggi-do, Republic of Korea

I. IDENTIFICATION OF THE MICROORGANISM
Identification reference given by the | Accession number given by the
DEPOSITOR: | INTERNATIONAL DEPOSITARY
Leuconostoc falkenbergense LBMB321010 | AUTHORITY:
 | KCTC 15613BP

II. SCIENTIFIC DESCRIPTION AND/OR PROPOSED TAXONOMIC DESIGNATION
The microorganism identified under I above was accompanied by:
[ ] a scientific description
[ ] a proposed taxonomic designation
(Mark with a cross where applicable)
III. RECEIPT AND ACCEPTANCE
This International Depositary Authority accepts the microorganism identified under I above,
which was received by it on September 14, 2023.
IV. RECEIPT OF REQUEST FOR CONVERSION
The microorganism identified under I above was received by this International Depositary
Authority on and a request to convert the original deposit to a deposit under the Budapest Treaty
was received by it on
V. INTERNATIONAL DEPOSITARY AUTHORITY
Name: Korean Collection for Type Cultures | Signature(s) of person(s) having the power
Address: Korean Collection for Type Cultures | to represent the International Depositary
of Korea Research Institute of Bioscience and | Authority or of authorized official(s):
Biotechnology (KRIBB) Jeonbuk Branch, 181, |
Ipsin-gil, Jeongeup-si, Jeollabuk-do 56212, | KIM, Song Gun
Republic of Korea | Date: September 14, 2023

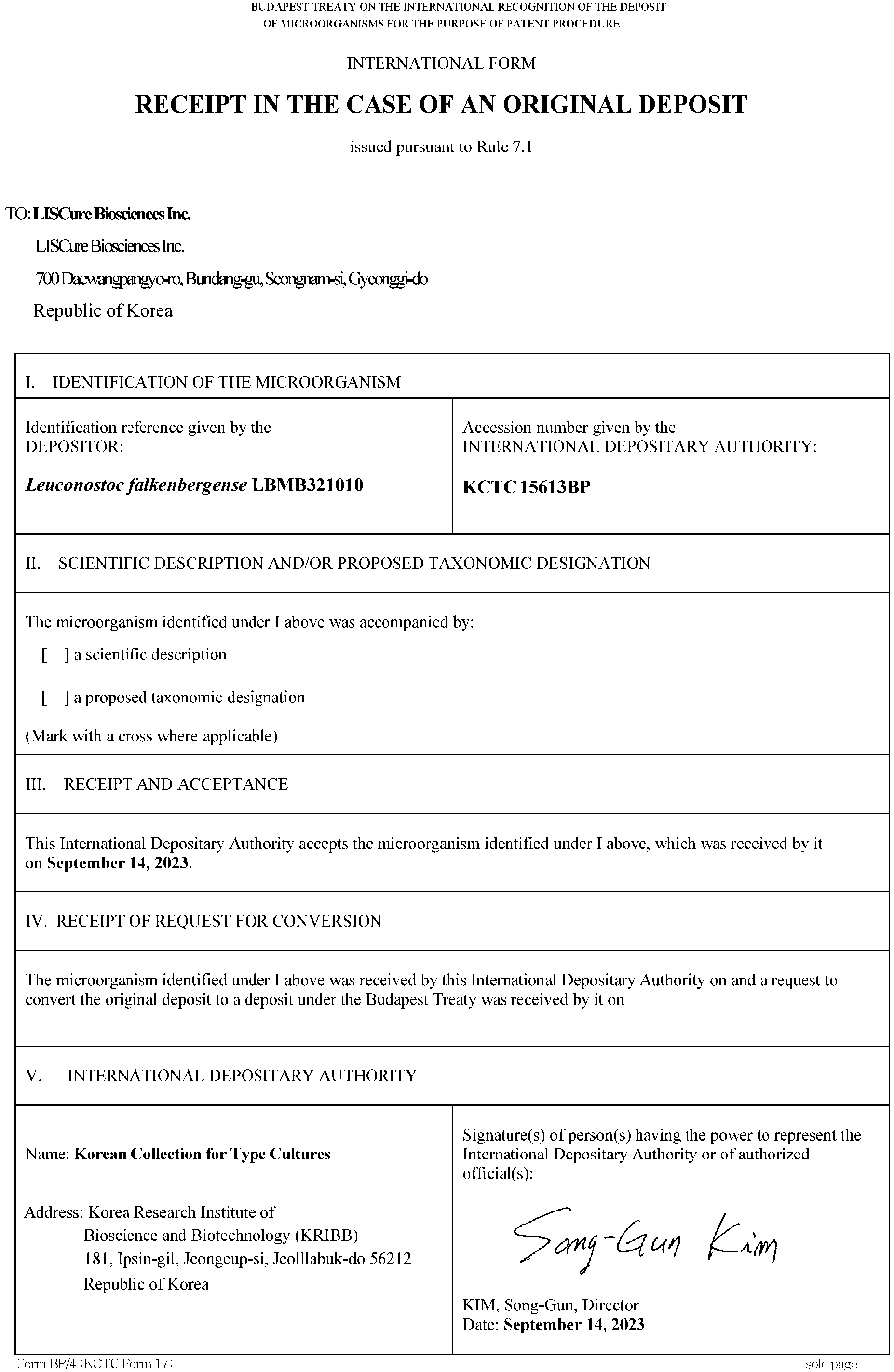
BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

RECEIPT IN THE CASE OF AN ORIGINAL DEPOSIT

issued pursuant to Rule 7.1

TO: LISCure Biosciences Inc.
LISCure Biosciences Inc.
700 Daewangpangyo-ro, Bundang-gu, Seongnam-si, Gyeonggi-do
Republic of Korea

I. IDENTIFICATION OF THE MICROORGANISM
Identification reference given by the
DEPOSITOR:

Leuconostoc falkenbergense LBMB321010 | Accession number given by the
INTERNATIONAL DEPOSITARY AUTHORITY:

KCTC 15613BP
II. SCIENTIFIC DESCRIPTION AND/OR PROPOSED TAXONOMIC DESIGNATION
The microorganism identified under I above was accompanied by:
[ ] a scientific description
[ ] a proposed taxonomic designation
(Mark with a cross where applicable)
III. RECEIPT AND ACCEPTANCE
This International Depositary Authority accepts the microorganism identified under I above, which was received by it
on September 14, 2023.
IV. RECEIPT OF REQUEST FOR CONVERSION
The microorganism identified under I above was received by this International Depositary Authority on and a request to
convert the original deposit to a deposit under the Budapest Treaty was received by it on
V. INTERNATIONAL DEPOSITARY AUTHORITY
Name: Korean Collection for Type Cultures

Address: Korea Research Institute of
Bioscience and Biotechnology (KRIBB)
181, Ipsin-gil, Jeongeup-si, Jeolllabuk-do 56212
Republic of Korea | Signature(s) of person(s) having the power to represent the
International Depositary Authority or of authorized
official(s):


KIM, Song-Gun, Director
Date: September 14, 2023
Form BP/4 (KCTC Form 17) sole page

## Claims

1. A *Leuconostoc falkenbergense* LBMB321010 (Accession number: KCTC15613BP) strain having excellent α-synuclein aggregation inhibitory activity.

2. A pharmaceutical composition for preventing or treating degenerative brain diseases, comprising the strain of claim 1 or a culture thereof as an active ingredient.

3. The pharmaceutical composition for preventing or treating degenerative brain diseases of claim 2, wherein the degenerative brain disease is any one disease selected from the group consisting of Parkinson's disease, Alzheimer's disease, Huntington's disease, amyotrophic lateral sclerosis, Creutzgeldt-Jakob disease, stroke, and multiple sclerosis.

4. The pharmaceutical composition for preventing or treating degenerative brain diseases of claim 2, wherein the composition is administered orally or parenterally.

5. The pharmaceutical composition for preventing or treating degenerative brain diseases of claim 4, wherein the parenteral administration includes intravenous injection, subcutaneous injection, intramuscular injection, intraperitoneal injection, intradermal administration, topical administration, intranasal administration, intrapulmonary administration, or intrarectal administration.

6. A food composition for preventing or alleviating degenerative brain diseases, comprising the strain of claim 1 or a culture thereof as an active ingredient.

7. The food composition for preventing or alleviating degenerative brain diseases of claim 6, wherein the food is a health functional food.

8. A food additive composition for preventing or alleviating degenerative brain diseases, comprising the strain of claim 1 or a culture thereof as an active ingredient.

9. A feed composition for preventing or alleviating degenerative brain diseases of animals, comprising the strain of claim 1 or a culture thereof as an active ingredient.

10. A feed additive composition for preventing or alleviating degenerative brain diseases of animals, comprising the strain of claim 1 or a culture thereof as an active ingredient.
